# EUROPEAN PATENT SPECIFICATION

(11) **EP 4 213 869 B1**
(45) Date of publication and mention of the grant of the patent: **25.11.2026**
(21) Application number: 21772845.0
(22) Date of filing: 17.09.2021
(51) Int. Cl.: A61K 38/24, A61P 15/08, A61P 5/06

(54) **NEW USE OF LUTEINISING HORMONE**
NEUE VERWENDUNG DES LUTEINISIERENDEN HORMONS
NOUVELLE UTILISATION DE L'HORMONE LUTÉINISANTE

(30) Priority: 18.09.2020 IT 202000022015
(43) Date of publication of application: 26.07.2023
(73) Proprietor: La Marca, Antonio, 41123 Modena (IT)
(72) Inventor: La Marca, Antonio, 41123 Modena (IT)
(74) Representative: Delbarba, Andrea
(86) International application number: PCT/IB2021/058493
(87) International publication number: WO 2022/058951

(56) References cited:
- WO-A1-00/67778
- WO-A1-2020/243679
- US-A1- 2005 049 199
- DOS SANTOS FERNANDA ARAUJO ET AL: "Follicular right shift: Xenografting queens' ovarian tissue into severe combined imunnodeficiency mice and its responses to exogenous gonadotropin", THERIOGENOLOGY, LOS ALTOS, CA, US, vol. 86, no. 7, 6 June 2016 (2016-06-06), pages 1811 - 1817, XP029725190, ISSN: 0093-691X, DOI: 10.1016/J.THERIOGENOLOGY.2016.05.034
- "Meeting abstracts", JOURNAL OF ASSISTED REPRODUCTION AND GENETICS, KLUWER ACADEMIC PUBLISHERS-PLENUM PUBLISHERS, NE, vol. 27, no. 5, 8 April 2010 (2010-04-08), pages 197 - 226, XP019820817, ISSN: 1573-7330
- MAGALHES-PADILHA D M ET AL: "Effect of sequential medium onculture of goat ovarian cortical tissue", ANIMAL REPRODUCTION SCIENCE, ELSEVIER SCIENCE PUBLISHERS, AMSTERDAM, NL, vol. 132, no. 3, 27 May 2012 (2012-05-27), pages 159 - 168, XP028504920, ISSN: 0378-4320, [retrieved on 20120607], DOI: 10.1016/J.ANIREPROSCI.2012.05.017
- BLUMENFELD ET AL: "Gonadotropin-releasing hormone agonist decreases chemotherapy-induced gonadotoxicity and premature ovarian failure in young female patients with Hodgkin lymphoma", FERTILITY AND STERILITY, ELSEVIER, AMSTERDAM, NL, vol. 89, no. 1, 1 January 2008 (2008-01-01), pages 166 - 173, XP022415954, ISSN: 0015-0282, DOI: 10.1016/J.FERTNSTERT.2007.02.010
- ALVIGGI CARLO ET AL: "Recombinant luteinizing hormone supplementation in assisted reproductive technology: a systematic review", FERTILITY AND STERILITY, ELSEVIER, AMSTERDAM, NL, vol. 109, no. 4, 10 April 2018 (2018-04-10), pages 644 - 664, XP085376881, ISSN: 0015-0282, DOI: 10.1016/J.FERTNSTERT.2018.01.003
- MIGNINI RENZINI MARIO ET AL: "Retrospective analysis of treatments with recombinant FSH and recombinant LH versus human menopausal gonadotropin in women with reduced ovarian reserve", JOURNAL OF ASSISTED REPRODUCTION AND GENETICS, PLENUM PUBLISHING, US, vol. 34, no. 12, 4 September 2017 (2017-09-04), pages 1645 - 1651, XP036376919, ISSN: 1058-0468, [retrieved on 20170904], DOI: 10.1007/S10815-017-1034-Z
- ALVIGGI CARLO ET AL: "Biological versus chronological ovarian age: implications for assisted reproductive technology", REPRODUCTIVE BIOLOGY AND ENDOCRINOLOGY, BIOMED CENTRAL LTD, GB, vol. 7, no. 1, 22 September 2009 (2009-09-22), pages 101, XP021063504, ISSN: 1477-7827, DOI: 10.1186/1477-7827-7-101
- PING-PING LV ET AL: "Role of anti-Müllerian hormone and testosterone in follicular growth: a cross-sectional study", BMC ENDOCRINE DISORDERS, BIOMED CENTRAL LTD, LONDON, UK, vol. 20, no. 1, 8 July 2020 (2020-07-08), pages 1 - 10, XP021279043, DOI: 10.1186/S12902-020-00569-6
- EDUARDO L MOTTA ET AL: "Human choriogonadotropin prior to controlled ovarian stimulation and in vitro fertilization improves implantation, and pregnancy rates", JOURNAL OF ASSISTED REPRODUCTION AND GENETICS, KLUWER ACADEMIC PUBLISHERS-PLENUM PUBLISHERS, NE, vol. 26, no. 6, 16 June 2009 (2009-06-16), pages 305 - 311, XP019730443, ISSN: 1573-7330, DOI: 10.1007/S10815-009-9322-X

## Description

### FIELD OF THE INVENTION

The present invention relates to luteinising hormone (LH) or a molecule having LH-like activity or a composition comprising it for use in inducing the activation, and/or the maturation or the development of primordial and/or primary follicles in an individual.

### PRIOR ART

The activation, growth and development of small immature follicles to follicles ready for ovulation is a slow process, which takes a few months and is characterized by a large loss of growing follicles. Thus, there is the need for hundreds of primordial follicles to be activated and grow to obtain ovulation of a single follicle.

Only the last stages of follicular growth can be influenced by hormones (i.e. FSH and LH), in particular, the last two weeks of the follicles life. These hormones act on the antral follicles (follicles larger than 2 millimetres that can be easily identified by ovarian ultrasound) allowing the simultaneous growth of those follicles available in the ovaries at that time and enabling a superovulation of multiple oocytes that thus contrasts with the natural selection and ovulation of a single follicle. This strategy, called ovarian stimulation, is the basis of in vitro fertilisation techniques.

A new strategy, based on the use of LH, is illustrated in the present invention. In fact, the invention relates to a new therapeutic scheme aimed at acting on the first phases of follicular growth. Indeed, the disclosure shows the possibility of increasing the activation of primordial follicles and the development of primary follicles in later development. With this new approach the number of antral follicles can be increased, and these follicles can be available for ovarian stimulation.

In a woman's ovary after puberty, several types of ovarian follicles are present at the same time: primordial, primary, secondary or antral and preovulatory.

The primordial follicles are microscopic structures characterised by a single-cell layer of granulosa cells surrounding the oocyte; they are generated during intrauterine life and then remain quiescent until they are activated.

Activation occurs through mechanisms that are still poorly understood, although it seems conceivable that various intraovarian factors may play a key role in the processes that regulate the activation of primordial follicles from their quiescent state.

These intraovarian factors are for example the cytokine *stem cell factor* (SCF), growth factors belonging to the neurotrophin family, *vascular endothelial growth factor* (VEGF), *bone morphogenetic protein* 4 (BMP-4), *leukaemia inhibitory factor* (LIF), *basic fibroblast growth factor* (FGF2) and *keratinocyte growth factor* (KGF) (Hsueh AJ, et al Endocr Rev. 2015 and Gershon E, et Int J Mol Sci. 2020).

In addition, a study conducted in transgenic mice showed that deletion of the gene encoding for the transcription factor forkhead box O3a (FOXO3a) causes activation of all primordial follicles and consequent depletion of the ovarian reserve, thus revealing that FOXO3a is the main regulator of the transition between the quiescent and activated state in primordial follicles (John GB, et al, Dev Biol. 2008).

To date, the molecular details of the full mechanism mediated by FOXO3a expression have not been fully elucidated, but it has been suggested that FOXO3a may act through the signal transduction pathway known as the phosphatidylinositol-3-kinase (PI3K) pathway. (John GB, et al, Dev Biol. 2008).

When a primordial follicle is activated, a process begins that in the best case can lead to ovulation in several months (but this process can take up to 6 months). The primordial follicle becomes primary and this leads to an increase in diameter and a thickening of the granulosa cells into a multilayer. Then a fluid-filled antrum appears within the follicle, where the secretions of the granulosa are found; in this state the follicle is referred to as a secondary or antral follicle. Along this path, most of the primordial follicles undergo a process of atresia and apoptosis; therefore, the number of antral follicles will be much lower than the number of primordial follicles that were activated to grow.

The human ovary contains between 266,000 and 472,000 primordial follicles. With increasing age, the population of primordial follicles decreases progressively, leading to an estimated menopausal stock of less than 100 to 1000 resting follicles. Age-related depletion of the resting follicle stock occurs due to two processes: atresia and entry into the growth phase. Although the number of follicles entering the growth phase may be modulated by pool size or endocrine factors, recent studies suggest multiple paracrine/autocrine factors acting elusively (Hsueh AJ, et al, Endocr Rev. 2015).

As follicles enter the growth phase, they enlarge, either through GC proliferation or an increase in oocyte size. A zona pellucida begins to be deposited around the oocyte shortly after the start of follicular growth; however, its exact cellular source remains unclear. Gradually, the follicles become secondary follicles (Gougeon A., Endocr Rev. 1996 ; Erickson GF, St al, Fertil Steril. 2001).

Taking into account that from the pre-antral stage the ovulatory follicle will pass through eight stages to reach the pre-ovulatory size, the time required for this follicle to pass from the pre-antral to the ovulatory stage takes more than 2 months ( Gougeon A., Endocr Rev. 1996).

In mammals, it has long been recognised that the majority (>99%) of follicles present at birth become atresic and only less than 1% reach ovulation. Thus, the 'normal' fate for each individual follicle is to disappear by entering atresia, a process that can be considered normal, allowing the ovary to produce cyclic ovulatory activity. In primates, atresia affects only early-growing follicles. (Dalbies-Tran R, et al, Cells. 2020). The average atresia rate is around 30 and 32% for pre-antral and early antral follicles, respectively. In contrast, atresia of larger follicles varies significantly during the cycle, being inversely correlated with circulating FSH levels (Gougeon A. et al, Endocr Rev. 1996 and Erickson GF et al. Fertil Steril. 2001).

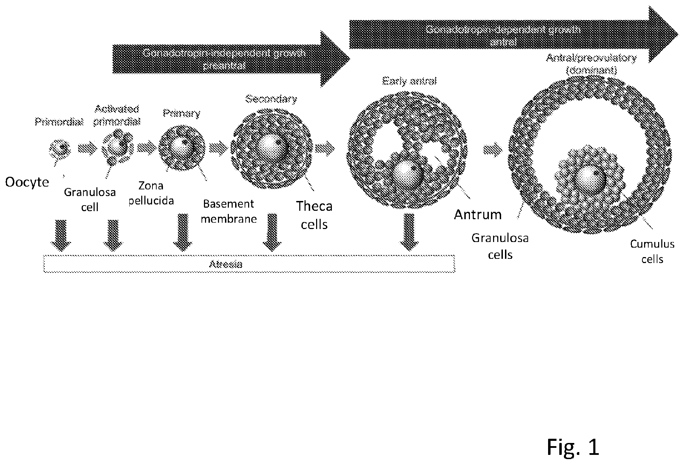

This part of folliculogenesis, from the primordial follicles to the small pre-antral follicle and described above, is called gonadotropin-independent (Fig. 1) because it is commonly believed that it is not controlled by follicle stimulating hormone (FSH) and LH under physiological conditions. So much so that it also occurs in patients without pituitary production of FSH or LH, i.e. women without a pituitary gland or women taking hormonal contraceptives. (Gougeon A. et al, Endocr Rev. 1996; Erickson GF et al. Fertil Steril. 2001).

When the follicles reach a more advanced stage of development, they are referred to as antral follicles and can be sub-millimetre in size but can also be larger, up to 9 mm. The population of antral follicles can therefore be recognised by ultrasound (when they are larger than 1-2 mm). In addition, antral follicles produce anti-Müllerian hormone (AMH), which is secreted into the bloodstream. An ultrasound scan for antral follicle counting (defined as ovarian follicles with a diameter of 2-10 mm) and/or AMH measurement therefore allows to measure the antral follicle pool. (Broekmans FJ, et al, Fertil Steril. 2010)
However, antral follicles are sensitive and dependent on gonadotropins.

From a size of 2 mm, the follicles become more dependent on FSH as their percentage of atresia decreases when FSH increases. (Gougeon A et al, 1984 Clinical Pathology of the Endocrine Ovary). These follicles have a low intrafollicular oestrogen-androgen ratio and androstenedione is the dominant steroid. Selectable antral follicles become more responsive to gonadotropins in terms of quality and growth rate, but their FSH-induced aromatase remains poorly expressed. During selection, the follicle destined for ovulation switches from an androgen-producing to an oestrogen-producing structure, expressing its FSH-induced aromatase activity (Gershon Eet allnt J Mol Sci. 2020).

Thus, gonadotropin-dependent folliculogenesis begins (Fig.1) and the antral follicles continue to grow only under the control of FSH. Of the antral follicles that are present monthly in the ovaries, the one that has the most receptors for FSH (i.e. the greatest sensitivity to FSH) is the follicle that will grow the fastest and most efficiently.

Around the time of dominant follicle selection, the expression of LH receptors can be detected in the follicular cells. LH stimulates progesterone secretion from luteal cells and androgen secretion, while FSH stimulates progesterone and oestradiol secretion from granulosa cells. During follicular growth, luteal androgens produced by LH are converted to oestrogen or bound to androgen receptors in the granulosa cells. In this way, androgens have been shown to increase the sensitivity of the follicle to FSH through up-regulation of the FSH receptor. (Luo W, et al, 2006 Biology of Reproduction).

The selected growing follicle will produce high quantity of steroids which, once introduced into the bloodstream, will lead to a relative and momentary reduction in pituitary FSH production. For the reason, in 99.5% of cases, a woman ovulates one oocyte at a time. High levels of oestradiol increase the sensitivity of the pituitary gland to hypothalamic GnRH, which may explain why in the middle of the cycle the pituitary gland discharges a high quantity of LH into the blood (LH peak). The preovulatory follicle, on stimulation of the endogenous secretion of pituitary LH into the blood (LH peak), ovulates, thus allowing the oocyte to be captured by the tubal ampulla and eventually fertilised by a spermatozoon.

It has been known for many decades that the hormone LH is involved in controlling (gonadotropin-dependent) folliculogenesis. Furthermore, a recombinant LH molecule, used precisely for ovarian stimulation, has been on the market for about 30 years. Ovarian stimulation is defined as a pharmacological treatment for inducing the development of ovarian follicles. It can be used for two purposes: 1) for timed intercourse or insemination; 2) in ART (Assisted Reproductive Technology), to obtain multiple oocytes at follicular aspiration (Zegers-Hochschild et al, 2017, ICMART Glossary). Ovarian stimulation is often performed by the administration of FSH with or without LH or LH activity. It acts upon the gonadotropin-dependent folliculgenesis, stimulating the growth of antral follicles present in the ovary, by administering an adequate amount of FSH (the doses can vary according to patient characteristics), possibly together with LH or LH activity (usually 75 or 150 units per day) for about two weeks. In the context of ovarian stimulation for ART treatment, the aim is to obtain the growth of all the antral follicles present, thus avoiding the natural process of selection and recruitment that would instead lead to the growth and ovulation of a single follicle.

Ovarian stimulation with FSH gonadotropin with or without LH or LH activity is the basis of ART treatment, as it enables a possibly high number of oocytes to be obtained for fertilisation. The number of oocytes obtained after ovarian stimulation depends on the number of antral follicles. The greater the number of antral follicles available, the greater the number of oocytes recruited. Since the number of retrieved oocytes is directly related to the number of embryos that can be created, this inevitably leads to an increase in the cumulative rate of pregnancy and live births per cycle of ovarian stimulation initiated (La Marca A, et al, Hum Reprod Update. 2014).

For these reasons, there is a great need to identify a therapy that can promote the activation and maturation of primordial and/or primary follicles to increase the reserve of antral follicles which, in turn, can mature and become mature oocytes after adequate ovarian stimulation.

As mentioned above, two phases during the ovarian folliculogenesis can be identified. Under physiological conditions, the first phase is gonadotropin-independent, while the second is gonadotropin-dependent. The ability to act therapeutically in the early stages of follicle development is very intriguing as it allows physicians to manipulate the ovarian reserve of patients and may lead to an increase in the number of antral follicles and AMH in women with a low basal ovarian reserve. Currently, there are no drugs approved for this indication (i.e. to activate/mature primordial and/or primary follicles during the gonadotropin-independent phase). Therefore, the possibility of using LH to activate and promote the growth of primordial and/or primary follicles, hitherto considered gonadotropin-independent, i.e. not sensitive to FSH or LH, is proposed as a new and effective therapeutic strategy to increase functional ovarian reserve.

As discussed above and illustrated more in detail below, the LH or molecule having LH-like activity is used according to this invention in a very different manner than in ovarian stimulation protocols with gonadotrophins stimulating gonadotropin-dependent folliculogenesis according to the prior art. Most importantly, the LH or molecule having LH-like activity is administered at a different stage of folliculogenesis, namely at the stage of activation and/or maturation of primordial and/or primary follicles, a development stage which under physiological conditions is gonadotrophin-independent. It is thus administered at an earlier stage of folliculogenesis than in classical ovarian stimulation protocols. Furthermore, the dose administered is between 150 and 450 International Units, thus much higher than the dose of 75 to 150 International Units applied in classical ovarian stimulation protocols.

WO00/67778 discloses the induction of folliculogenesis in anovulatory women, including the administration of luteinising hormone or biologically-active analogue thereof.

Alviggi et al, discusses recombinant hLH supplementation which appears to be beneficial in two subgroups of patients: 1) women with adequate prestimulation ovarian reserve parameters and unexpected hyporesponse to rhFSH monotherapy; and 2) women 36-39 year of age (Fertility and Sterility vol. 109, no. 4, 2018, pages 644-664).

Furthermore, the duration of treatment according to this invention is at least 30 days whereas ovarian stimulation protocols apply LH for 14 days only. Moreover, according to the uses and methods of this invention, LH or a molecule having LH-like activity is not administered concomitantly with FSH, while such concomitant administration is mandatory in ovarian stimulation protocols.

### SUMMARY OF THE INVENTION

A first aspect of the present invention relates to luteinising hormone (LH) or a molecule having LH-like activity for use as defined in the appended claims.

A second aspect of the present invention relates to a composition comprising LH or a molecule having LH-like activity for the medical uses described above. In one embodiment, the composition comprises salts, buffers, surfactants, excipients, carriers, preservatives and/or combinations thereof accepted for the preparation of pharmaceutical products. The composition is preferably formulated in liquid form, preferably in the form of a sterile solution, emulsion, or suspension, or else it is in powder form, preferably lyophilised, so as to be reconstituted to obtain a liquid formulation.

A third aspect of the present dislcosure relates to a method for inducing the activation and/or maturation of primordial and/or primary follicles in an individual. Said method comprises at least a step of administering an effective amount of LH or of a molecule having LH-like activity or of a composition comprising it to an individual who has a need for it.

### BRIEF DESCRIPTION OF THE FIGURES

Figure 1 shows a diagram of human gonadotropin-independent and gonadotropin-dependent folliculogenesis;
Figure 2 shows the number of (A) primordial and (B) primary follicles (mean per histological section ± SEM) in the original ovarian cortex or after two days of culture without (control) or with LH. Within every graph * denotes statistical significance versus the original biopsies whilst ** denotes significance versus the original biopsies and the controls (two-way ANOVA; P < 0.05; n = 6 cultures, 11-18 sections examined per sample);
Figure 3 shows a representation of the proportion of follicles in the different stages of maturation in ovarian cortex samples of the original biopsies, after two days of culture without LH or with LH. The distribution of the follicles is shown as a percentage of the total viable follicles. * denotes statistical significance in relation to the original biopsies whilst ** shows significance versus both the original biopsies and the controls (p < 0.05);
Figure 4 shows the increases in the fraction of oocytes that show cytoplasmic translocation of FOXO3a;
Figure 5 shows the relative expression of CCN genes in primary cultures of ovarian tissue incubated for 48 hours with LH. Significant differences between the samples treated with LH and the controls are marked with * (P < 0.005);
Figure 6 shows the image of an immunoblot relating to a membrane representative of the expression of genes of the CCN family following stimulation with LH;
Figure 7 shows the correlation between the basal LH and the percentage increase in AMH.

### DEFINITIONS

In the context of the present invention, the term "International Unit" (IU) as used here means a unit of measurement of the amount of a substance, based on its effect or on its biological activity.

In the context of the present invention, the term "ultrasound count of antral follicles" or "AFC" means the count of antral follicles in an individual obtained by means of a transvaginal pelvic ultrasound scan.

In the context of the present invention, the term "ovarian reserve" means the number and/or quality of oocytes, reflecting the ability to reproduce. Ovarian reserve can be assessed by any of several means. They include: female age; number of antral follicles on ultrasound; anti-Mullerian hormone levels; follicle stimulating hormone and oestradiol levels; clomiphene citrate challenge test; response to gonadotropin stimulation, and oocyte and/or embryo assessment during an ART procedure, based on number, morphology or genetic assessment of the oocytes and/or embryos..

In the context of the present invention, "CCN" means a family of extracellular matrix-associated proteins involved in intercellular signalling. Because of their dynamic role within the ECM they are considered matricellular proteins.

### DETAILED DESCRIPTION OF THE INVENTION

A first aspect of the present invention relates to luteinising hormone (LH) or a molecule having LH-like activity for use in inducing the activation and/or maturation or development of primordial and/or primary follicles in an individual having a reduced ovarian reserve.

The molecule having LH-like activity is selected from the group consisting of chorionic gonadotropin (CG) or an LH-agonist. Examples of LH agonists are drug-like low-molecular-weight ligands that interact allosterically within the seven transmembrane domains of the LH/CG receptors as thienopyrimidines and other pharmacological chaperones (pharmacoperones).

In fact, the Applicant has demonstrated that the administration of LH or of a molecule having LH-like activity is capable of stimulating the process leading to the activation of primordial follicles, or their maturation into primary follicles. The maturation of primordial follicles is in fact associated with an increase in primary follicles and a decrease in primordial follicles.

In one embodiment, the activation and/or maturation or development of primordial and/or primary follicles is associated with an increase in the ovarian reserve of the individual. The increase in the ovarian reserve is preferably defined by means of an ultrasound count of antral follicles (AFC) and/or by means of a serum assay of anti-Müllerian hormone (AMH). The Applicant has demonstrated that the administration of LH or of a molecule having LH-like activity is capable of increasing the ovarian reserve in an individual, with a consequent increase in the AFC values and in the serum levels of AMH.

Furthermore, contrary to the prior art, the applicant has demonstrated that LH or a molecule having LH-like activity, is capable of stimulating the first phase of folliculogenesis, known as gonadotropin-independence.

Table 1 shows the differences between the present invention and the use of LH for ovarian stimulation, i.e., for stimulation of the gonadotropin-dependent phase.

**Table 1**

| | **Inducing the activation, and/or the maturation of primordial and/or primary follicles (Present invention)** | **Ovarian stimulation to promote folliculogenesis (Prior art)** |
|---|---|---|
| time | Before ovarian stimulation | During ovarian stimulation |
| duration | at least 30 days | 14 days |
| dose | 150-450 U | 75-150 IU |
| Concomitant treatment with FSH | Not allowed | Mandatory |

Preferably, the individual is a human subject of the female sex.

In one embodiment of the description, the individual has an ovarian reserve within the norm. Preferably, the individual has an AFC value greater than 10, more preferably greater than 12 and/or the individual has a serum concentration of AMH greater than 2 ng/ml, more preferably greater than 2.5 ng/ml.

For the purpose of the invention, the individual has a reduced ovarian reserve, namely, the individual has an AFC value lower than 10, more preferably lower than 8 and/or the individual has a serum concentration of AMH lower than 2 ng/ml, more preferably lower than 1.5 ng/ml.

In one embodiment, said LH is human LH (hLH) or said CG is human chorionic gonadotropin (hCG); preferably, said LH is recombinant human LH (rhLH) or extracted from urine or said CG is recombinant human chorionic gonadotropin (rhCG).

Said LH or said CG is preferably used as a recombinant protein or purified/isolated.

In this context, reference is preferably made to the use of the entire LH or CG protein, or to homologues, analogues, variants, derivatives or fragments of the LH or CG protein on condition that the activity of LH or CG is maintained.

In one embodiment, said LH or CG is a biologically active homologue. In one embodiment of the invention, the protein variants of LH or CG to which reference is made have modifications in the N-terminal and/or C-terminal regions, for example adapted to increase the activity of LH or CG. Said modifications are preferably selected from among deletions, additions, alterations of amino acids and combinations thereof. Alternatively, said LH or CG can be modified, preferably in its primary structure, by acetylation, carboxylation, glycosylation, phosphorylation and combinations thereof.

In a further embodiment, said LH or CG is conjugated/bound to a molecule, a metal, or a marker, for example proteins, for the preparation of fusion proteins. In a further embodiment of the invention, said LH or CG is modified by means of molecular biology techniques to improve its resistance to proteolytic degradation and/or to optimise its solubility or to improve its pharmacokinetic characteristics. In a further embodiment, said LH or CG, preferably the protein form, is conjugated to at least one molecule capable of improving its stability and/or its half-life and/or its solubility in water and/or its immunological characteristics. Said molecule, by way of example, is polyethylene glycol (PEG).

In a further embodiment of the invention, said LH or CG protein is synthesised by means of conventional protein synthesis techniques known to the skilled person. For example, the protein can be synthesized by chemical synthesis using solid-phase peptide synthesis. Alternatively, LH or CG can be produced with recombinant DNA techniques known to the person skilled in the art.

In a further embodiment of the invention, the LH or CG protein, after the synthesis thereof or production thereof with recombinant DNA techniques, is isolated or purified with methods known to the person skilled in the art. For example, LH or CG can be purified with chromatographic methods (gel-filtration, ion exchange and immunoaffinity), by means of high-performance liquid chromatography (HPLC, RP-HPLC, ion exchange HPLC, size-exclusion HPLC) or by precipitation (immunoprecipitation).

Said LH or the molecule having LH-like activity is used, for the medical purposes stated above, in an amount comprised between 150 and 450 International Units (IU) per day, more preferably between 170 and 350 IU per day, even more preferably comprised between 180 and 250 IU per day.

In one embodiment, LH or the molecule having LH-like activity is taken at least once a day, preferably at least twice a day. For the medical purposes described above, LH or the molecule having LH-like activity is taken for a period of time comprised between 40 and 120 days, more preferably comprised between 50 and 100 days. In a preferred embodiment, LH or the molecule having LH-like activity is taken per at least 60 days. Preferably, LH or the molecule having LH-like activity is administered parenterally, preferably subcutaneously or intramuscularly.

LH or the molecule having LH-like activity is administered in association or in combination with an infertility treatment, preferably in association or in combination with ovarian stimulation.

LH or the molecule having LH-like activity is preferably administered before the infertility treatment, preferably before ovarian stimulation.

In one preferred embodiment, LH or the molecule having LH-like activity is administered neither in association nor in combination with FSH.

A second aspect of the present invention relates to a composition comprising LH and/or a molecule having LH-like activity for the medical uses described above. In one embodiment, the composition comprises salts, buffers, excipients, carriers, preservatives and/or combinations thereof accepted for the preparation of pharmaceutical products.

In one embodiment, the composition is formulated for parenteral administration, preferably for subcutaneous or intramuscular administration. The composition is preferably formulated in liquid form, preferably in the form of a sterile solution, emulsion or suspension, or else it is in powder form, preferably lyophilised, so as to be reconstituted to obtain a liquid formulation. In a preferred embodiment, the composition is formulated as a powder, preferably lyophilised, so as to be reconstituted to obtain a liquid formulation.

In one embodiment of the invention, said composition is formulated for enteral administration, preferably for oral administration. In particular, the composition is formulated in solid form, preferably in the form of lozenges, capsules, tablets, granular powder, hard-shelled capsules, orally dissolving granules, sachets or pills.

A third aspect of the present disclosure relates to a method for inducing the activation and/or the maturation or development of primordial and/or primary follicles in an individual. Said method comprises at least a step of administering an effective amount of LH or of a molecule having LH-like activity or of a composition comprising it to an individual who has a need for it.

In one embodiment, the activation and/or maturation of primordial and/or primary follicles is associated with an increase in the ovarian reserve of the individual. The increase in the ovarian reserve is preferably defined by means of an ultrasound count of antral follicles (AFC) and/or by means of a serum assay of anti-Müllerian hormone (AMH).

Preferably, the individual is a human subject of the female sex.

In one embodiment of the disclosure, the individual has an ovarian reserve within the norm. The individual preferably has an AFC value greater than 10, more preferably greater than 12 and/or the individual has a serum concentration of AMH greater than 2 ng/ml, more preferably greater than 2.5 ng/ml.

In one embodiment of the disclosure, the individual has a reduced ovarian reserve. The individual preferably has an AFC value lower than 10, more preferably lower than 8 and/or the individual shows a serum concentration of AMH lower than 2 ng/ml, more preferably lower than 1.5 ng/ml.

In one embodiment, the method for inducing the activation and/or the maturation of primordial and/or primary follicles in an individual is associated or combined with a method for treating infertility in an individual.

LH or the molecule having LH-like activity is taken in association or in combination with an infertility treatment in an individual, preferably in association or in combination with ovarian stimulation.

LH or the molecule having LH-like activity is preferably taken before the infertility treatment, preferably before ovarian stimulation.

In one preferred embodiment, LH or the molecule having LH-like activity is taken on its own and not in association with FSH.

### EXAMPLE

### In vitro study

In mammals, the ovaries contain a limited and finite number of oocytes organised in primordial follicles. The majority of primordial follicles are maintained quiescent as a reserve for the duration of reproductive life. Only a few of them are activated and develop into more advanced follicular stages. Although the molecular mechanisms that regulate the maintenance of dormancy and the activation of primordial follicles have not been completely understood, various studies have demonstrated that they depend on coordinated actions of inhibiting/activating molecules produced by the oocyte and the communication of the oocyte itself with somatic and intraoocyte cells.

For these reasons, the aims of this study are to investigate a) whether luteinising hormone (LH) can promote the cytoplasmic-nuclear translocation of FOXO3a and b) whether LH can be capable of activating members of the CCN family in in-vitro cultures of ovarian tissue.

### Materials and methods

### Ovarian tissue donors

2x2x2 mm³ pieces of ovarian cortical tissue were obtained from biopsies taken from patients undergoing diagnostic laparoscopy and salpingochromoscopy as a workup in the study of couple infertility. The patients enrolled in this study historically had a regular menstrual cycle and stated that they had no pathologies at the time of sampling. All patients were asked for and granted written consent to the use of the samples and of the clinical data for research purposes.

### Tissue preparation

The ovarian tissue was obtained under conditions of sterility and transferred to a Petri dish containing 1X PBS, where the medulla tissue was separated from the cortex by using a scalpel. The ovarian cortical tissue was further divided into smaller pieces with a size of 1 mm³. A single piece deriving from the initial biopsy was directly fixed in 4% paraformaldehyde at 4 °C overnight for the immunohistochemical analysis. A single piece deriving from the initial biopsy was instead treated for total RNA extraction with the method described below. The remaining pieces were deposited in a 12-well *multiwell* plate (one piece per well) and covered with a pre-balanced culture medium (500 µl/well; 1 hour at 37 °C). The culture medium was composed of 25 mM HEPES, 1 mM penicillin/streptomycin, 1 mM L-glutamine, 1 mM amphotericin B, and 10% foetal bovine serum (FBS) dissolved in the basic medium McCoy 5A. All the reagents were purchased from Sigma-Aldrich (St. Louis, MO, USA). The samples were cultured at 37 °C under conditions of a humidified atmosphere containing 5% CO2 for 24 hours before the treatments.

### Treatments

After 24 hours of primary culture, the samples were treated with 10 µM of LH (dissolved in the culture medium) for an additional 48 hours (one change of medium after the first 24 hours). The control samples were cultured only in the medium without LH. The samples thus treated were processed for total RNA extraction or for the immunohistochemical analysis as described below.

### RT-qPCR

The single piece of ovary deriving from the original biopsy and the samples treated with 10 µM of LH (and the corresponding controls) were directly lysed in 1 ml of Tri-Reagent^{®} (Sigma Aldrich) and immediately processed for total RNA extraction according to the protocol described by the manufacturer. The extracted RNA was placed in 20 µl *RNase-free water* and digested with DNase I (Promega, Madison, WI, USA). The purified RNA was quantified using a Nanodrop ND-1000 spectrophotometer (Thermo Fisher Scientic, Waltham, MA, USA). The messenger RNA corresponding to every sample was reverse transcribed into cDNA using M-MuLV reverse transcriptase (NEB, Ipswich, MA, USA). The cDNAs were used as a template for RT-qPCR reactions.

For every gene considered, the samples were evaluated in triplicate and expressed as a mean in order to allow an analysis of gene expression. The results were normalised using the expression of the β-actin gene expressed constitutively. The negative controls of the reactions amplified distilled water in place of the cDNA. The genes evaluated were: CCN2, CCN3 and CCN5. The relative expression of every gene was calculated by applying the 2^{-ΔΔCt} method described by Livak and Schmittgen, 2001.

### Slide preparation

The samples deriving from the original biopsy, the samples treated with LH (and the corresponding control samples) were fixed in 4% paraformaldehyde at 4 °C overnight. The samples were subsequently dehydrated with an ascending alcohol series and embedded, after clearing in xylene, in blocks of paraffin. Serial slices of ovary were obtained by cutting with a microtome (7 µm) and used for immunohistochemical analysis or morphological staining (haematoxylin and eosin) to enable a count of the follicles.

### Immunohistochemistry

The paraffin was removed from the slides with xylene and they were rehydrated with a descending alcohol series. The slides were unmasked to allow the exposure of nuclear antigens. The slides were treated with the primary anti-FOXO3a or anti-p-FOXO3a antibody (mouse anti-human; 1:100) and subsequently incubated with a secondary antibody conjugated to TRITC or FITC (goat anti-mouse; 1:10000). All of the antibodies used were purchased from Santa Cruz, CA, USA. The samples were counterstained with DAPI to enable identification of the nuclei. A double-blind count was made of the positive follicles by two different operators who randomly selected at least 5 fields per slide.

### SDS-PAGE

The proteins contained in the individual samples were extracted with RIPA buffer and quantified with the Bradford assay. A same amount of proteins (50 µg) was mixed with 2X loading buffer (4% SDS; 20% glycerol; 0.004% bromophenol blue; 0.125 M Tris-HCl at pH 6.8; 10% 2-mercaptoethanol), 1X protease inhibitor cocktail and 5 mM phenylmethylsulfonyl fluoride (PMSF, Sigma Aldrich, St. Louis, MO, USA) and finally boiled for 10 minutes to linearize the proteins. The protein extracts were resolved by means of a 12% polyacrylamide/bis-polyacrylamide gel under denaturing conditions. The ColorBurst Electrophoresis Marker (Sigma Aldrich, St. Louis, MO, USA) was used as a molecular weight marker. The samples were loaded in triplicate in three identical gels. The proteins were run for 90 minutes at a constant voltage (120 V).

### Immunoblot

The proteins separated on the basis of molecular weight were transferred using the Trans-Blot SD semi-dry transfer cell (Bio-Rad, Hercules, CA, USA) on three membranes (one per gel) made of polyvinylidene fluoride (PVDF, Thermo scientific, Rockford, IL, USA). The membranes were blocked overnight in a solution of Tris-buffered saline (TBS, pH 7.4) and 5% partially skimmed milk (skim milk powder, Sigma Aldrich, St. Louis, MO, USA). The membranes were subsequently incubated for 1 hour individually with anti-CCN2, -CCN3 or -CCN5 antibody (Santa Cruz Biotechnology, Dallas, TX, USA) diluted 1:200. After three washes with 0.05% Tween 20 (Sigma Aldrich, St. Louis, MO, USA) in TBS, the membranes were further incubated with the rabbit anti-goat secondary antibody, horseradish peroxidase (HRP) conjugated (Bethyl, Montgomery, TX, USA) 1:10000. Finally, the membranes were covered with ECL Western Blotting Substrate (Thermo Scientific, Rockford, IL, USA) and the bands were detected by chemiluminescence using ChemiDoc XRS+ (Bio-Rad, Hercules, CA, USA). The signals were acquired and semi-quantified by means of a system for the analysis of digital images (VersaDoc Imaging System and QuantityOne software, Bio-Rad Laboratories Inc.). After the acquisition of the images, the membranes were washed and rehybridised with the anti-human β tubulin primary antibody (Abcam, Cambridge, UK) obtained in rabbits diluted 1:500 and processed as described previously. After development, the intensity of the β tubulin bands was used as an internal control on the initially loaded quantity of proteins.

### Statistical analysis

A statistical analysis was conducted on the relative expressions obtained with RT-qPCR by combining the Kruskal-Wallis test followed by Dunn-Bonferroni's test (P<0.005). A statistical analysis of the bands after immunoblotting was conducted using GraphPad Prism software and applying a two-way ANOVA test, considering a probability P<0.05 as significant. For the count of ovarian follicles, the number of follicles was compared within the treated groups using an ANOVA test followed by a post hoc test. Differences with a probability P < 0.05 were considered significant.

### Results

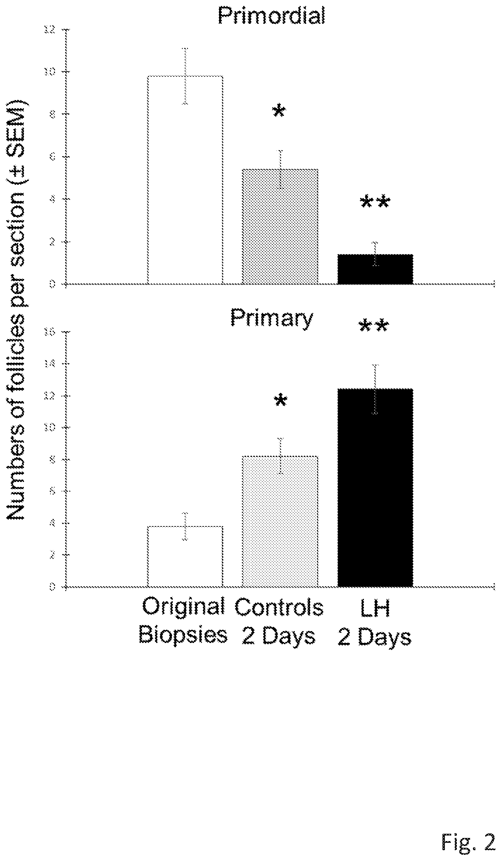

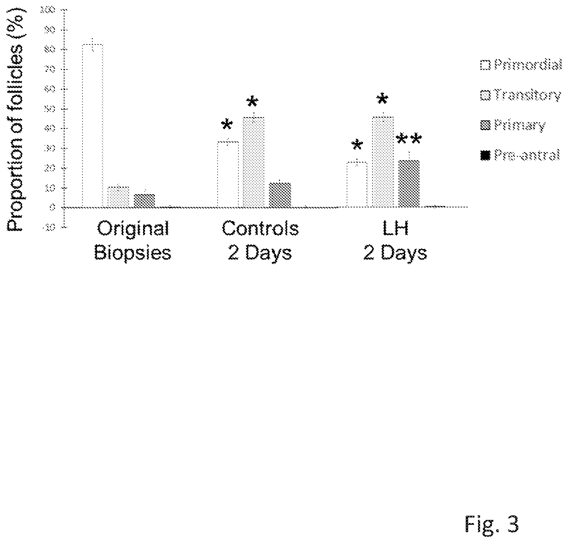

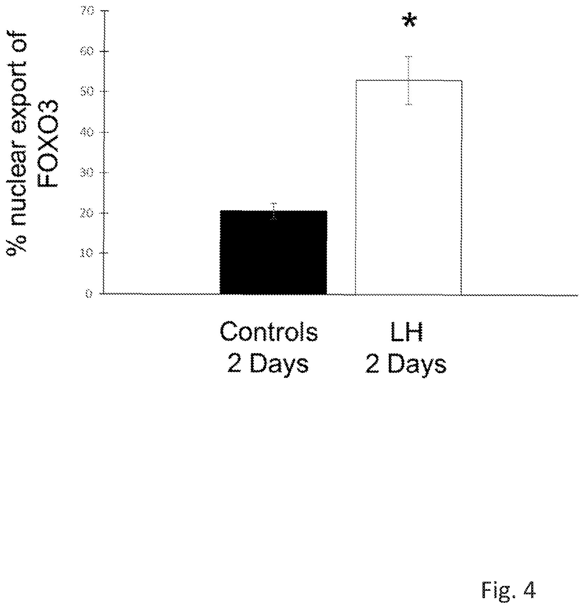

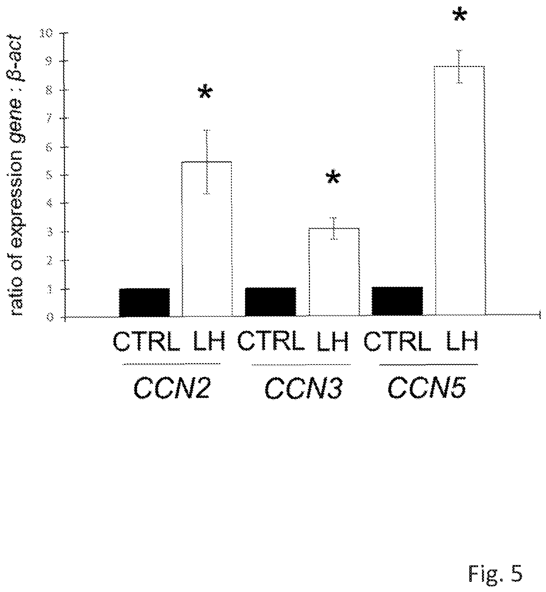

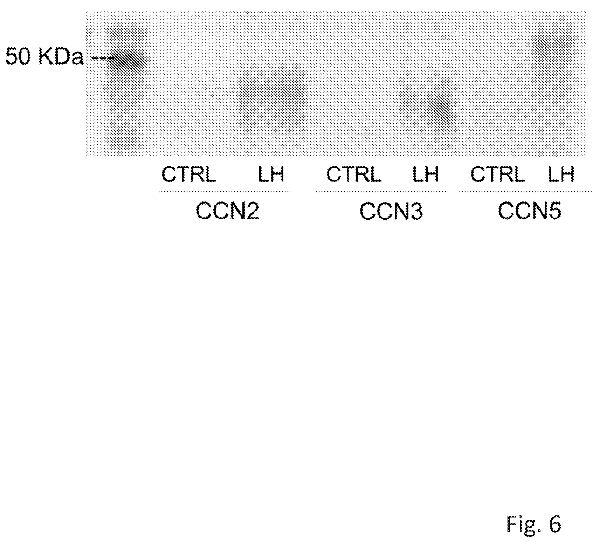

As shown in Figure 2, the number of primary follicles was increased by the treatment with LH (with a consequent decrease in the number of primordial follicles). A well-documented phenomenon of spontaneous activation of primordial follicles following the culturing of ovarian tissue is also present in the experiments. In fact, the controls (ovarian tissue in culture without LH) show a significant increase in the number of primary follicles compared to those present in the original biopsy. LH revealed to be capable of increasing the number of the primary follicles versus both the controls and the original biopsies. Figure 3 shows the percentage of follicles in the various stages of maturation in relation to the total present in the initial biopsy. The changes in the percentages of the various subpopulations were significant both in the control samples and in the samples treated with LH. LH showed to be effective in increasing not only the number of follicles which, on passing through the phenotype transition, reach the primary follicle stage, but also the number of the follicles that progress beyond this phase to reach the pre-antral phenotype. Figure 4 summarises the percentage of oocytes showing to be positive at the cytoplasmic level compared to the percentage of oocytes wherein FOXO3a was localised in the nucleus. LH induced the activation of primordial follicles by promoting the translocation of FOXO3a from the nucleus to the cytoplasm. LH was capable of inducing the relative expression of all three genes and of the proteins belonging to the CCN family after two days of treatment, confirming that the phosphatidylinositol-3-kinase pathway was activated (Figures 5 and 6).

### In vivo study

A prospective pilot trial was conducted on 30 patients affected by idiopathic infertility. The aim of the study was to investigate the effects of a medium/long-term treatment with exogenous LH on the ovarian reserve, namely the number of antral follicles (AFC) and the circulating AMH. The hypothesis behind this study was that LH may activate or promote the growth of primordial and/or primary follicles hence leading to an increase in preantral and antral follicles which may be recognized in vivo by ultrasound (the antral follicle count-AFC) or by measuring AMH (the hormone produced by the antral follicles).

The age of the enrolled patients ranged from 18 to 40 years. Exclusion criteria were: presence of known infertility factors (endocrine-ovulatory factor, tubal factor, endometriosis), presence of ovarian cysts, presence of endocrine/metabolic disorders, and male factor. The demographic characteristics of the patients are shown in table 2. All of the patients had regular menstrual cycles (every 28-35 days).

**Table 2**

| | Patient characteristics (n=30) | |
|---|---|---|
| Age (M±SD) | 32±5 | |
| BMI | 22±2.5 | |
| Duration of infertility (months) | 18±9 | |
| Primary infertility (%) | 90% | |
| Average duration of cycle (days) | 29±2 | |
| AMH (ng/ml) | 1.6±0.6 | |
| D3FSH (IU/L) | 6.5±1.9 | |
| D3E2 (pg/ml) | 45±23 | |
| D3 LH (IU/L) | 4.5±2.6 | |
| AFC (n) | 12±4.4 | |

The 30 patients in the study were treated with LH at a dosage of 187.5 U per day (75 U in the morning and 112.5U in the evening) for two months. They underwent an analysis of the ovarian reserve by means of an assay of serum AMH and an ultrasound count of antral follicles (AFC) at time 0, after one month and at the end of the therapy (2 months). Of the 30 patients, 11 had undergone a cycle of in vitro Fertilization (IVF) in a time interval ranging from 10 days to 90 days before the start of the therapy with LH. These patients repeated a second cycle of IVF after the two months of therapy with LH.

### Aims of the study

Primary aim of the study: to evaluate the effect of LH administration on ovarian reserve markers, namely AFC and AMH.

Secondary aim: to evaluate the predictive criteria of ovarian response to the LH therapy. To evaluate the outcome of IVF cycles before and after the long-lasting therapy with LH.

### Results of the study

It emerged from the study that, after treatment with LH for two months, there was an average 60% increase in AFC and 52% increase in AMH (Table 3)

**Table 3. Ovarian reserve markers after one and two months of therapy**

| | Basal | 1st month of therapy | 2nd month of therapy |
|---|---|---|---|
| AMH (ng/ml) (mean ± SD) | 1.6 (±0,61) | 2.1 (±0,92) | 2.43 (±1.2) |
| % change in AMH | Na | 31% | 52% |
| AFC (mean ± SD) | 12 (± 4,4) | 15 (±7.7) | 19.2 (± 8,4) |
| % change in AFC | Na | 25% | 60% |

Of the 30 patients enrolled, 12 showed an extremely reduced ovarian reserve (defined as AMH <1ng/ml and/or AFC <7). In these patients the response to the therapy was comparable to that found in the patients with a normal ovarian reserve.

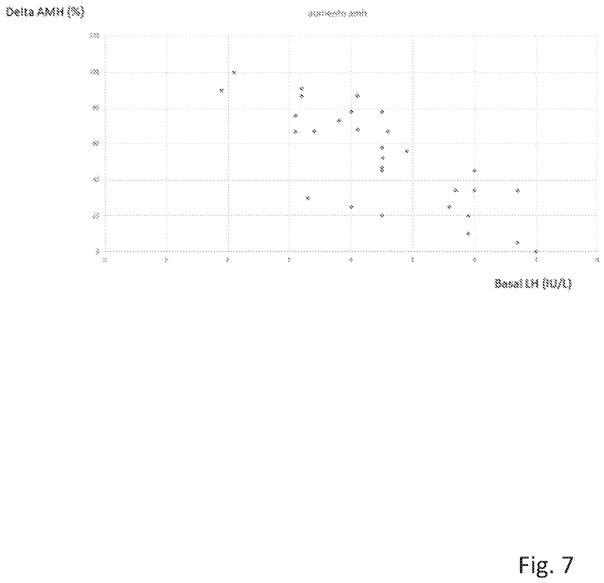

Using multiple regression analysis, we then investigated which variables can predict a positive response to therapy with LH and it emerged that a younger age and low serum levels of FSH and LH are the criteria predictive of a good response to therapy with exogenous LH. In particular, the percentage increase in AMH was greater when the age was lower and the basal plasma levels of LH were lower (Figure 7).

**Table 4: Predictors of the Increase in AMH after therapy with LH**

| Dependent Y | Increase in AMH |
|---|---|
| Analytical | Enter |
| Sample size | 30 |
| Coefficient of Determination R² | 0.7226 |
| R²-adapted | 0.7021 |
| Multiple correlation coefficient | 0.8501 |
| Residual standard deviation | 15.3146 |

### Regression Equation

| **Independent variables** | **Coefficient** | **Std. Error** | **t** | **P** |
|---|---|---|---|---|
| **(Constant)** | 185.61452 | | | |
| **age** | -2.68351 | 0.86582 | -3.099 | 0.0045 |
| **LH** | -10.24955 | 2.99391 | -3.423 | 0.0020 |

### Analysis of variance

| **Source** | **DF** | **Sum of squares** | **Mean squares** |
|---|---|---|---|
| **Regression** | 2 | 16497.6808 | 8248.8404 |
| **Residual** | 27 | 6332.5261 | 234.5380 |
| **F-Ratio** | 35.1706 | | |
| **Significance** | P<0.001 | | |

### Zero-order correlation coefficients

| **Variable** | **R** |
|---|---|
| **age** | -0.776 |
| **LH** | -0.790 |

### Pre-treatment with LH and IVF outcome

Some of the recruited patients had undergone a cycle of ovarian stimulation in the 3 months preceding the therapy with LH, without achieving pregnancy. These patients underwent a second IVF cycle after two months of therapy with LH. The comparison between the two ovarian stimulations is shown in table 5. It emerged that the treatment with LH led to an increase in the embryos obtained. In particular, it enabled more blastocysts to be obtained and more patients had cryopreserved embryos after the ovarian stimulation treatment.

**Table 5**

| | Cycle before the LH treatment | Cycle after the LH treatment |
|---|---|---|
| N | 11 | 11 |
| Age | 34±5 | 34±5 |
| Basal AMH (ng/ml) | 1.2±0.3 | 1.7±0.5 |
| AFC (n) | 10±4 | 14±6 |
| Starting dose of FSH | 2251U | 2251U |
| % patients with d2/d3 embryos | 91 % | 64% |
| % patients with d5 embryos | 9 % | 36% |
| Average number of cleavage stage embryos available for transfer | 2±1 | 4±2 |
| Number of blastocysts available for transfer | 1±0 | 2±1 |
| % of patients who have supernumerary embryos | 18% | 54% |
| Clinical pregnancy | 0 | 36% |

## Claims

1. Luteinising hormone (LH) or a molecule having LH-like activity or a composition comprising it, for use in inducing the activation, and/or the maturation or development of primordial and/or primary follicles in an individual having a reduced ovarian reserve, wherein the molecule having LH-like activity is selected from the group consisting of chorionic gonadotropin (CG) or an LH-agonist, wherein LH or the molecule having LH-like activity is administered in association or in combination with an infertility treatment for a period of time comprised between 40 and 120 days in an amount comprised between 150 and 450 International Units (IU) per day, wherein the individual has a reduced ovarian reserve, defined as a serum concentration of AMH lower than 2 ng/ml and wherein the individual has a reduced ovarian reserve, defined as AFC lower than 10.

2. LH or a molecule having LH-like activity or a composition comprising it for use according to claim 1, in increasing primary follicles.

3. LH or a molecule having LH-like activity or a composition comprising it for use according to claim 1 or 2, in increasing the ultrasound count of antral follicles (AFC) and in increasing the serum values of anti-Müllerian hormone (AMH).

4. LH or a molecule having LH-like activity or a composition comprising it for use according to any one of claims 1-3, wherein the individual has a reduced ovarian reserve, defined as a serum concentration of AMH lower than 1.5 ng/ml.

5. LH or a molecule having LH-like activity or a composition comprising it for use according to any one of claims 1-4, wherein the individual has a reduced ovarian reserve, defined as AFC lower than 8.

6. LH or a molecule having LH-like activity or a composition comprising it for use according to any one of claims 1-5, wherein LH is human LH (hLH), preferably recombinant human LH (rhLH).

7. LH or a molecule having LH-like activity or a composition comprising it for use according to any one of claims 1-6, wherein CG is human CG (hCG), preferably recombinant human CG (rhCG).

8. LH or a molecule having LH-like activity or a composition comprising it for use according to any one of claims 1-7, wherein said LH is administered in an amount comprised between 170 and 350 IU per day.

9. LH or a molecule having LH-like activity or a composition comprising it for use according to any one of claims 1-8, wherein said LH is administered in an amount comprised between 180 and 250 IU per day.

10. LH or a molecule having LH-like activity or a composition comprising it for use according to any one of claims 1-9, wherein LH or the molecule having LH-like activity is taken for a period of time comprised between 50 and 100 days.

11. LH or a molecule having LH-like activity or a composition comprising it for use according to any one of claims 1-10, wherein LH or the molecule having LH-like activity is taken for a period of time of at least 60 days.

12. LH or a molecule having LH-like activity or a composition comprising it for use according to any one of claims 1-11, wherein LH or the molecule having LH-like activity is administered in association or in combination with ovarian stimulation.

13. LH or a molecule having LH-like activity or a composition comprising it for use according to any one of claims 1-12, wherein LH or the molecule having LH-like activity is administered before the infertility treatment

14. LH or a molecule having LH-like activity or a composition comprising it for use according to claim 13, wherein LH or the molecule having LH-like activity is administered before ovarian stimulation.

15. LH or a molecule having LH-like activity or a composition comprising it for use according to any one of claims 1-14, wherein LH or the molecule having LH-like activity is administered neither in association nor in combination with FSH.

## Patentansprüche

1. Luteinisierendes Hormon (LH) oder ein Molekül mit LH-ähnlicher Aktivität oder eine Zusammensetzung, die es umfasst, zur Verwendung bei der Induktion der Aktivierung und/oder der Reifung oder Entwicklung von primordialen und/oder primären Follikeln bei einer Person mit verminderter Eizellreserve, wobei das Molekül mit LH-ähnlicher Aktivität aus der Gruppe ausgewählt ist, die aus Choriongonadotropin (CG) oder einem LH-Agonisten besteht, wobei LH oder das Molekül mit LH-ähnlicher Aktivität in Assoziation oder in Kombination mit einer Behandlung gegen Unfruchtbarkeit über einen Zeitraum zwischen 40 und 120 Tagen in einer Menge zwischen 150 und 450 Internationalen Einheiten (IE) pro Tag verabreicht wird, wobei die Person eine verminderte Eizellreserve aufweist, die als eine AMH-Serumkonzentration von weniger als 2 ng/ml definiert ist, und wobei die Person eine verminderte Eizellreserve aufweist, die als AFC von weniger als 10 definiert ist.

2. LH oder ein Molekül mit LH-ähnlicher Aktivität oder eine Zusammensetzung, die es umfasst, zur Verwendung nach Anspruch 1 zur Erhöhung primärer Follikel.

3. LH oder ein Molekül mit LH-ähnlicher Aktivität oder eine Zusammensetzung, die es umfasst, zur Verwendung nach Anspruch 1 oder 2, zur Erhöhung der Ultraschallzählung der Antralfollikel (AFC) und zur Erhöhung der Serumwerte des Anti-Müller-Hormons (AMH).

4. LH oder ein Molekül mit LH-ähnlicher Aktivität oder eine Zusammensetzung, die es umfasst, zur Verwendung nach einem der Ansprüche 1-3, wobei die Person eine verminderte Eizellreserve aufweist, die als eine AMH-Serumkonzentration von weniger als 1,5 ng/ml definiert ist.

5. LH oder ein Molekül mit LH-ähnlicher Aktivität oder eine Zusammensetzung, die es umfasst, zur Verwendung nach einem der Ansprüche 1-4, wobei die Person eine verminderte Eizellreserve aufweist, die als AFC von weniger als 8 definiert ist.

6. LH oder ein Molekül mit LH-ähnlicher Aktivität oder eine Zusammensetzung, die es umfasst, zur Verwendung nach einem der Ansprüche 1-5, wobei es sich beim LH um humanes LH (hLH), vorzugsweise rekombinantes humanes LH (rhLH), handelt.

7. LH oder ein Molekül mit LH-ähnlicher Aktivität oder eine Zusammensetzung, die es umfasst, zur Verwendung nach einem der Ansprüche 1-6, wobei es sich beim CG um humanes CG (hCG), vorzugsweise rekombinantes humanes CG (rhCG), handelt.

8. LH oder ein Molekül mit LH-ähnlicher Aktivität oder eine Zusammensetzung, die es umfasst, zur Verwendung nach einem der Ansprüche 1-7, wobei das LH in einer Menge zwischen 170 und 350 IE pro Tag verabreicht wird.

9. LH oder ein Molekül mit LH-ähnlicher Aktivität oder eine Zusammensetzung, die es umfasst, zur Verwendung nach einem der Ansprüche 1-8, wobei das LH in einer Menge zwischen 180 und 250 IE pro Tag verabreicht wird.

10. LH oder ein Molekül mit LH-ähnlicher Aktivität oder eine Zusammensetzung, die es umfasst, zur Verwendung nach einem der Ansprüche 1-9, wobei LH oder das Molekül mit LH-ähnlicher Aktivität über einen Zeitraum zwischen 50 und 100 Tagen eingenommen wird.

11. LH oder ein Molekül mit LH-ähnlicher Aktivität oder eine Zusammensetzung, die es umfasst, zur Verwendung nach einem der Ansprüche 1-10, wobei LH oder das Molekül mit LH-ähnlicher Aktivität über einen Zeitraum von mindestens 60 Tagen eingenommen wird.

12. LH oder ein Molekül mit LH-ähnlicher Aktivität oder eine Zusammensetzung, die es umfasst, zur Verwendung nach einem der Ansprüche 1-11, wobei LH oder das Molekül mit LH-ähnlicher Aktivität in Assoziation oder in Kombination mit einer Stimulation der Eierstöcke verabreicht wird.

13. LH oder ein Molekül mit LH-ähnlicher Aktivität oder eine Zusammensetzung, die es umfasst, zur Verwendung nach einem der Ansprüche 1-12, wobei LH oder das Molekül mit LH-ähnlicher Aktivität vor der Behandlung gegen Unfruchtbarkeit verabreicht wird.

14. LH oder ein Molekül mit LH-ähnlicher Aktivität oder eine Zusammensetzung, die es umfasst, zur Verwendung nach Anspruch 13, wobei LH oder das Molekül mit LH-ähnlicher Aktivität vor der Stimulation der Eierstöcke verabreicht wird.

15. LH oder ein Molekül mit LH-ähnlicher Aktivität oder eine Zusammensetzung, die es umfasst, zur Verwendung nach einem der Ansprüche 1-14, wobei LH oder das Molekül mit LH-ähnlicher Aktivität weder in Assoziation noch in Kombination mit FSH verabreicht wird.

## Revendications

1. Hormone lutéinisante (LH) ou molécule présentant une activité similaire à celle de la LH, ou composition la comprenant, destinée à être utilisée pour induire l'activation et/ou la maturation ou le développement de follicules primordiaux et/ou primaires chez un individu présentant une réserve ovarienne réduite, dans laquelle la molécule présentant une activité similaire à celle de la LH est choisie dans le groupe constitué par la gonadotrophine chorionique (CG) ou un agoniste de la LH, dans laquelle la LH ou la molécule présentant une activité similaire à celle de la LH est administrée en association ou en combinaison avec un traitement de l'infertilité pendant une période comprise entre 40 et 120 jours, à une dose comprise entre 150 et 450 unités internationales (UI) par jour, dans laquelle l'individu présente une réserve ovarienne réduite, définie par une concentration sérique d'AMH inférieure à 2 ng/ml, et dans laquelle l'individu présente une réserve ovarienne réduite, définie par un CFA inférieur à 10.

2. LH ou molécule présentant une activité similaire à celle de la LH, ou composition la comprenant, destinée à être utilisée selon la revendication 1, pour augmenter les follicules primaires.

3. LH ou molécule présentant une activité similaire à celle de la LH, ou composition la comprenant, destinée à être utilisée selon la revendication 1 ou 2, pour augmenter le comptage des follicules antraux (CFA) mesurés par ultrasons et pour augmenter les taux sériques d'hormone anti-müllérienne (AMH).

4. LH ou molécule présentant une activité similaire à celle de la LH, ou composition la comprenant, destinée à être utilisée selon l'une quelconque des revendications 1-3, dans laquelle l'individu présente une réserve ovarienne réduite, définie par une concentration sérique d'AMH inférieure à 1,5 ng/ml.

5. LH ou molécule présentant une activité similaire à celle de la LH, ou composition la comprenant, destinée à être utilisée selon l'une quelconque des revendications 1-4, dans laquelle l'individu présente une réserve ovarienne réduite, définie par un CFA inférieur à 8.

6. LH ou molécule présentant une activité similaire à celle de la LH, ou composition la comprenant, destinée à être utilisée selon l'une quelconque des revendications 1-5, dans laquelle la LH est la LH humaine (hLH), de préférence la LH humaine recombinante (rhLH).

7. LH ou molécule présentant une activité similaire à celle de la LH, ou composition la comprenant, destinée à être utilisée selon l'une quelconque des revendications 1-6, dans laquelle la CG est une CG humaine (hCG), de préférence une CG humaine recombinante (rhCG).

8. LH ou molécule présentant une activité similaire à celle de la LH, ou composition la comprenant, destinée à être utilisée selon l'une quelconque des revendications 1-7, dans laquelle ladite LH est administrée à une dose comprise entre 170 et 350 UI par jour.

9. LH ou molécule présentant une activité similaire à celle de la LH, ou composition la comprenant, destinée à être utilisée selon l'une quelconque des revendications 1-8, dans laquelle ladite LH est administrée à une dose comprise entre 180 et 250 UI par jour.

10. LH ou molécule présentant une activité similaire à celle de la LH, ou composition la comprenant, destinée à être utilisée selon l'une quelconque des revendications 1-9, dans laquelle la LH ou la molécule présentant une activité similaire à celle de la LH est prise pendant une période comprise entre 50 et 100 jours.

11. LH ou molécule présentant une activité similaire à celle de la LH, ou composition la comprenant, destinée à être utilisée selon l'une quelconque des revendications 1-10, dans laquelle la LH ou la molécule présentant une activité similaire à celle de la LH est prise pendant une période d'au moins 60 jours.

12. LH ou molécule présentant une activité similaire à celle de la LH, ou composition la comprenant, destinée à être utilisée selon l'une quelconque des revendications 1-11, dans laquelle la LH ou la molécule présentant une activité similaire à celle de la LH est administrée en association ou en combinaison avec une stimulation ovarienne.

13. LH ou molécule présentant une activité similaire à celle de la LH, ou composition la comprenant, destinée à être utilisée selon l'une quelconque des revendications 1-12, dans laquelle la LH ou la molécule présentant une activité similaire à celle de la LH est administrée avant le traitement de l'infertilité.

14. LH ou molécule présentant une activité similaire à celle de la LH, ou composition la comprenant, destinée à être utilisée selon la revendication 13, dans laquelle la LH ou la molécule présentant une activité similaire à celle de la LH est administrée avant la stimulation ovarienne.

15. LH ou molécule présentant une activité similaire à celle de la LH, ou composition la comprenant, destinée à être utilisée selon l'une quelconque des revendications 1-14, dans laquelle la LH ou la molécule présentant une activité similaire à celle de la LH n'est administrée ni en association ni en combinaison avec la FSH.
